# EUROPEAN PATENT APPLICATION

(11) **EP 1 120 159 A1**
(43) Date of publication of application: **01.08.2001**
(21) Application number: 99203668.1
(22) Date of filing: 05.11.1999
(51) Int. Cl.: B01J 31/02, B01J 37/02, C07C 2/68

(54) **Supported ionic liquids, process for preparation and use thereof**

(71) Applicant: Hölderich, W. F., Prof., 67227 Frankenthal (DE)
(72) Inventor: DeCastro-Moreira, Christovao Paulo, 52072 Aachen (DE); Hoelderich, Prof. Wolfgang F., 67227 Frankenthal (DE); Sauvage, Emmanuelle, 34280 Carnon (FR); Valkenberg, Michael H., 52064 Aachen (DE)
(74) Representative: Schalkwijk, Pieter Cornelis, c.s.

(57) **Abstract**

The invention relates to a process to make a supported ionic liquid, the supported ionic liquid so obtained, and the use of this supported ionic liquid as a catalyst in chemical processes. The process involves the pretreatment of a dried support with a metal halide and impregnation of the pretreated support with an ionic liquid. The supported ionic liquid show less leaching of the ionic liquid when used in subsequent processes such as olefin alkylation. In the examples MCM-41 is used as catalyst carrier.

## Description

The present invention relates to improved supported ionic liquids and a process to make such improved supported ionic liquids, as well as to their use as a catalyst in chemical processes.

Supported ionic liquids are know from, for instance, EP-A-0 553 009 and US 5,693,585. EP-A-0 553 009 discloses how a calcined porous support is impregnated with an ionic liquid to make a supported ionic liquid. The same procedure is followed in US 5,693,585. Both references disclose that the supported ionic liquid is used as a catalyst in alkylation with an olefin.

However, the supported catalysts as disclosed therein were found to suffer from two serious drawbacks. First of all, the calcined support and the ionic liquid used in the impregnation step must be shielded from water with great care, since any traces of water that are present during the impregnation step will result in deterioration of the support. Secondly, in the chemical process wherein the supported ionic liquid is used as a catalyst, the ionic liquid was found to leach from the support at an unacceptably high rate. Hence, there is a need for improved supported ionic liquids and an improved process to make them.

Surprisingly, we have found that a specific pretreatment step of the support, prior to the impregnation step, can be used to overcome most of the known difficulties.

More particularly, the process according to the invention comprises the steps of:
- pretreatment, wherein a dried support is contacted with a metal halide in an inert atmosphere and in the absence of ionic liquid, and
- impregnation, wherein an ionic liquid is contacted with the pretreated support in an inert atmosphere.

It is noted that the term "dried support" as used throughout this document is meant to encompass all support materials, as presented in more detail below, which are essentially free of water. Such dried supports can be obtained by any suitable technique, e.g. calcination, desiccation, and the like. Depending on the chemical structure of the support, calcining may be the preferred way of drying the support. Supports based on silica, alumina, aluminosilicate, such as zeolite and mesoporous materials of the MCM type, and the like, are preferably dried by calcination. The calcination temperature is not critical, and what temperatures can be applied will again depend on the chemical structure of the support. Typically, calcination is performed at temperatures in the range of 300-650°C, preferably 450-600°C, for three hours in order to render supports suitable for use according to the invention. The pore size of the dried support is not critical. To keep the supports dry, they are to be stored in an inert atmosphere.

It is furthermore to be understood that the term "impregnation" as used relates to any technique wherein an ionic liquid is absorbed in a support and/or an ionic liquid is adsorbed on the surface of said support. Typically, such impregnation simply means that the pretreated support and the ionic liquid are blended. However, the ionic liquid might be formed *in situ* during the impregnation step, meaning that the pretreated support is mixed with the chemicals to form the ionic liquid.

The process steps are to be performed in an inert atmosphere to prevent water from entering. Although the process according to the invention is less sensitive to water, in that the support is not destroyed if water is present in the pretreatment step, the water will react with the other chemicals used in the process, thereby wasting raw materials and resulting in the undesired formation of by-products. Preferably, nitrogen or argon is used to ensure an inert atmosphere, nitrogen being preferred. However, dried inert solvents can also be used.

The support materials that can be used in the process according to the invention include microporous and mesoporous as well as macroporous supports. Hence the support may have an average pore diameter from 3 nm to 1 mm, as can be determined using conventional techniques. Preferably, the supports have a BET surface area of 0.1-1500 m²/g, more preferably 100-1200 m²/g, and most preferably 100-400 m²/g.
Suitable and preferred supports include clays and metal oxides, such as silica, alumina, aluminosilicates, especially zeolites (such as zeolite Y ex Degussa), titanium oxide, boron oxide, or any other metal oxide containing hydroxyl groups on the surface. Such supports include the preferred MCM-types of materials that have a desirable high surface area and include mesoporous materials such as MCM 41. The process of the invention is most advantageous for making supported ionic liquids on a "regularly ordered" or "structured" support, hereinafter also called a nanosupport, such as zeolites and MCM-type materials. Such structured/ordered supports show sharp peaks in the XRD spectrum, as is known in the art.

The metal halide used in the pretreatment step can be any conventional metal halide. Preferably, the metal halide is selected from the metal halides suitable for making an ionic liquid. Preferably, the metal halides are selected from aluminium, gallium, iron, copper, zinc, indium, and tin halides. More preferred metal halides are aluminium, iron, and tin halides. Preferred halides are the chlorides. Most preferred is aluminium trichloride.

The ionic liquid used to impregnate the support can be any conventional ionic liquid. Typically, they are classified as fused salt compositions that are liquid at a temperature below the melting point of the individual components. Preferably, the melting point of the ionic liquids as used in the process of the invention is between -10 and 100°C, more preferably -10-60°C, and most preferably 0-30°C, all at atmospheric pressure.

Conventional ionic liquids are typically formed by combining a metal halide and an organic base. Suitable metal halides are those compounds that can form anions containing polyatomic halide bridges in the presence of a hydrocarbyl-containing amine hydrohalide salt. Preferably, the metal halides are covalently bonded halides of metals of Groups 8-14 of the Periodic Table. Preferred metals are aluminium, gallium, iron, copper, zinc, tin, and indium, with aluminium being most preferred. The metal halide used to make the ionic liquid may be the same as or different from the metal halide used in the pretreatment step of the catalyst. Preferably, the metal halide of the ionic liquid is the same compound as used in the pretreatment step. Examples of suitable metal halides include copper monochloride, ferric trichloride, zinc dichloride, and aluminium trichloride. Organic bases suitable for forming conventional ionic liquids include hydrocarbyl-containing amine hydrohalide salts, such as alkyl-containing amine hydrohalide salts based on trimethylamine, ethylenediamine, ethylenetriamine, morpholine, imidazole, guanidine, picoline, piperazine, pyridine, pyrazole, pyrrolidine, triazine, triazole, pyrimidine, derivatives of such molecules, and/or mixtures thereof, and phosphonium compounds.

As is known in the art, various ratios of metal halide to organic base can be used to make the conventional ionic liquids. Stoichiometric amounts of base and metal halide are defined such that a neutral ionic liquid is obtained. For use according to the invention, the ionic liquid preferably contains less than the desired amount of metal halide, this in order to compensate for the amount of metal halide that is present in and on the treated support. If the supported ionic liquid of the invention is to be used as a catalyst in subsequent alkylation reactions, the final ionic liquid must be acidic.

Ionic liquids that can be used in the process of the invention include chloroaluminates (such as the salts obtained by combining AlCl₃ and an organic base), chlorogallates (based on, e.g., GaCl₃), ternary ionic liquids (e.g., based on AlCl₃, (alkyl)imidazolium or (alkyl)pyridinium chloride, and a hydrocarbyl-substituted quaternary ammonium halide or hydrocarbyl-substituted phosphonium halide).

The pretreatment step is typically conducted by stirring a slurry of support and dried methylene chloride wherein metal halide is dissolved for about an hour at room temperature (25°C) in an inert atmosphere. The methylene chloride can be dried in a conventional way, e.g. by distillation over CaCl₂. Other solvents and reaction conditions can be chosen, as will be clear to the skilled person. If AlCl₃ is used, for instance, it is know that protic solvents cannot be used. However, most solvents can be used if SnCl₂ is used in the pretreatment step. The amount of solvent to be used depends on the reaction conditions. Typically, the solvent is used in an amount that allows proper stirring of the mixture. After said treatment, the solvent can be removed or the solution can be used as is. Removed solvent can be recycled.

Depending on the support that is used, the ratio of metal halide to support in the pretreatment step needs to be optimized. Preferably, the metal halide reacts with the reactive groups, typically being hydroxyl groups, on the surface of the support. Therefore, the metal halide is preferably used in a more than stoichiometric amount, based on the amounts and types of reactive groups on said surface. For a dried Y-zeolite, 1 g of FeCl₃ was successfully used to treat 5 g of the zeolite. However, the metal halide can be used in far greater amounts, especially if the same metal halide is used to make the ionic liquid *in situ* during the subsequent impregnation step.
As stated above, the current process tolerates supports that are not fully "dried supports." However, preferably dried supports are used to avoid wasting metal halide through the formation of HCI. When using dried supports, the amount of metal halide needed to treat the support can be significantly reduced.

In a preferred embodiment of the invention, a support is first contacted with metal halide and subsequently with ionic liquid. If so desired, the pretreated support can be impregnated with said ionic liquid immediately after the pretreatment step, preferably in the same reactor. As explained below, the ionic liquid may be added in the ionic liquid form or may be formed *in situ* by adding the compounds forming such ionic liquid. In the latter case, the metal halide used to form the ionic liquid may all have been present already during the pretreatment step (requiring just the addition of organic base during the impregnation step). Because this mode of operation allows for a high metal halide concentration in the pretreatment step, with consequent shorter reaction times, this is the preferred way to conduct the process of the invention. However, it is also feasible to first treat with a certain amount of metal halide and to subsequently add, in any sequence, organic base and further metal halide. The metal halide so used may or may not be the same compound.

The amount of ionic liquid for impregnating the treated support will also depend on the support used and the amount of pretreatment agent on the support, as is explained in more detail below. Good results were obtained in processes where an excess of ionic liquid was used, in particular where the weight ratio of ionic liquid to support was chosen from 2:1 to 1:2. Preferably, so much ionic liquid is used that after stirring for 0.5 hour still some unabsorbed ionic liquid can be seen on the surface of the support. If an excess of ionic liquid is used, the excess is preferably removed to avoid leaching of ionic liquid upon use of the supported catalyst formed. Suitably, the excess ionic liquid is removed by soxhlet extraction with refluxing methylene chloride. The ionic liquid so removed can be reused for the impregnation of fresh support.

The impregnation can be conducted by stirring a mixture of the pretreated support and ionic liquid or by stirring a mixture of pretreated support and compounds capable of making an ionic liquid *in situ.* Said stirring is preferably conducted above the melting temperature of the ionic liquid. Stirring for a period of at least three hours, preferably overnight, was found to suffice in most cases. However, the skilled person will have no problem varying the impregnation conditions, if so desired. For instance, it is possible to use a solvent to improve the homogeneity of the supported ionic liquid.

When analyzed by X-ray diffraction (XRD), the supported ionic liquids so obtained showed that after impregnation the support was still intact. Although the inventors do not wish to be bound by the following theory, it is believed that the beneficial properties of the pretreatment step are due to the fact that the metal halide will react with reactive groups, particularly OH-groups, of the support under formation of HCI. Preferably, the reaction results in a support to which the metal halide is covalently bonded. Said HCI is liberated and will diffuse from the support before the ionic liquid is introduced. If a conventional impregnation technique is used, the ionic liquid will react with the reactive groups of the support, under the formation of HCI. In this case, however, an ionic liquid is present and the HCI that is formed will have super-acidic properties, as is known in the art, see, for instance, "Chemistry of non-aqueous solutions: Current progress," Chapter 5 of R.T. Carlin, J.S. Wilkes; Ed. G. Mamantov, I. Popov, *Chemistry and Speciation in Chloroaluminate Molten Salts* Wiley-VCH, NY, 1994, pp. 277-306. Hence, conventional impregnation techniques are expected to result in the formation of super-acidic HCI, whereas this is prevented in the process of the invention. In the case of structured/ordered supports, such as zeolites and MCM-type materials, super-acidic HCI typically was found to destroy the support material. Accordingly, conventional impregnation processes result in (partially) destroyed supports, while in the process according to the invention this is not the case, or at least is the case to a lesser extent. Furthermore, the metal halide now attached to the support, particularly when it is covalently bonded to the support, will become part of the ionic liquid when the ionic liquid is absorbed on and into the support. This, together with the fact that the structure of the support is not damaged, is believed to be the reason why the supported ionic liquid according to the invention shows less leaching of the ionic liquid than conventional supported ionic liquids do when used in subsequent processes.

The supported ionic liquids so obtained are pre-eminently suited for use as a catalyst in chemical processes. Examples of such processes include, but are not limited to: the alkylation of aromatic compounds, such as benzene, naphthalene, phenanthrene, and the like, with olefins, such as ethylene, propylene, isobutene, decene, dodecene, and the like; the oligomerization of olefins, such as ethylene and propylene; the acylation of olefins and aromatic compounds with acid chlorides and/or anhydrides, such as acetyl chloride and acetic anhydride; carbonylation reactions, such as the reaction of phenol with carbon dioxide; and oxidation reactions, such as the process to produce benzoic acid from toluene. Such use is known in the prior art. However, the ionic liquid was found to leach from pretreated supports at a much lower rate than from supports not so pretreated. Hence, the supported ionic liquids of the invention have a longer catalyst life and lead to lower contamination of the product stream than conventional supported ionic liquids.

The invention is elucidated by the following examples

### Experimental

MCM 41 was prepared as described by J. Beck et.al. in US 5,145,816 and *J. Am. Chem. Soc.* 1992, 114, 834.
FeCl₃ ex Fluka, > 99% purity
Butyl-methyl-imidazolium chloride ex Elementis Specialties
Ultra stable Y-zeolite ex Grace Co.
Methylene chloride ex Merck
Toluene ex Merck
Hexene ex Fluka
All solvents are >98% pure and dried using conventional techniques.

### Example 1

Under argon, 5 g of MCM 41 were stirred with a solution of 1g FeCl₃ (6.3 mmol) in 30 ml methylene chloride for 1 hour at room temperature. Methylene chloride was subsequently evaporated under reduced pressure to give a treated support.

The treated support was stirred at room temperature with 2.5 g (6.7 mmol) of an ionic liquid consisting of butyl-methyl-imidazolium chloride and FeCl₃ in a molar ratio of 1:1.22 for three hours under inert gas. The formed supported ionic liquid was freed of excess ionic liquid by soxhlet extraction with methylene chloride under reflux for a period of 24 hours. Methylene chloride was removed under vacuum to give the desired supported ionic liquid.

XRD spectra showed that the order/structure of the support did not deteriorate in this process.

### Comparative Example A

Example 1 was repeated, except that the treatment with the FeCl₃ solution in methylene chloride was omitted. XRD spectra of the supported ionic liquid obtained showed a severe deterioration of the support.

### Example 2

The process of Example 1 was repeated using ultra-stable Y-zeolite instead of MCM 41. XRD analysis of the fresh support and the formed supported ionic liquid showed that the support had not deteriorated in the process.

### Example 3

A coiled tubular reactor (diameter of the tube 6 mm, length 1 metre) with a frit near the downstream end carrying 1 g of the supported ionic liquid of Example 2 as a catalyst was placed in an oven.
At a temperature of 150°C, and with a weight hourly space velocity (WHSV) of 4h⁻¹, toluene was alkylated with 1-hexene. The process was conducted as a continuous gas phase reaction using a molar ratio of toluene to hexene of 10:1. The conversion, based on 1-hexene, dropped from 86% at the beginning of the reaction to around 45% after 3 hours. Thereafter, the conversion remained at the 45% level. A selectivity ranging from 65 to 85% was observed for the monoalkylated product. The other product was a mixture of hexene isomers and di-, or higher, alkylated product.

## Claims

1. Process to make a supported ionic liquid involving the steps of:
a) pretreating a, preferably dried, support with a metal halide in an inert atmosphere
b) impregnating the pretreated support with an ionic liquid in an inert atmosphere.

2. A process according to claim 1 wherein a solvent is used in step a) which is selected from CCl₄, CHCl₃, CH₂Cl₂, CH₃Cl, 1,2-dichloroethane, 1,1,2-trichloroethane, and fluorinated hydrocarbons.

3. A process according to claim 1 or 2 wherein the ionic liquid is used in an amount greater than the amount that can be absorbed/adsorbed by the support and wherein the excess is subsequently removed, preferably by (Soxhlet) extraction.

4. A process according to any one of the preceding claims wherein the ionic liquid is formed from the same metal halide as is used in the pretreatment step.

5. A process according to any one of the preceding claims wherein the ionic support is formed *in situ* during the impregnation step by the reaction of a metal halide and an organic base.

6. A process according to claim 5 wherein at least 10% by weight of the metal halide used to form the ionic liquid *in situ* in step b) is already present in pretreating step a).

7. A process according to any one of the preceding claims wherein the metal halide is selected from the group consisting of aluminium, gallium, iron, copper, zinc, indium, and tin halides.

8. A process according to any one of the preceding claims wherein the support is a nanosupport.

9. A supported ionic liquid obtainable by the process of any one of claims 1-8.

10. The supported ionic liquid of claim 9 wherein a regularly ordered support is used.

11. The supported ionic liquid of claim 9 or 10 wherein the untreated support has OH groups on the surface that react with the metal halide in step a) to form a treated support with covalently bonded metal halide moieties.

12. Use of a supported ionic liquid according to any of claims 9-11 as a catalyst in a chemical process.

13. Use according to claim 12 wherein the chemical process is alkylation with an olefin.
